Europäisches Patentamt

European Patent Office

Office européen des brevets

(11) Numéro de publication: **0 309 404**
**A2**

## DEMANDE DE BREVET EUROPEEN

(21) Numéro de dépôt: **88810626.7**

(22) Date de dépôt: **14.09.88**

(51) Int. Cl.⁴: **C 08 F 220/06**
C 08 F 6/00, A 61 K 9/22,
A 61 L 15/03

(30) Priorité: **25.09.87 CH 3726/87**

(43) Date de publication de la demande:
**29.03.89 Bulletin 89/13**

(84) Etats contractants désignés:
**CH DE FR GB IT LI**

(71) Demandeur: **BATTELLE MEMORIAL INSTITUTE**
**7 route de Drize**
**CH-1227 Carouge/Genève (CH)**

(72) Inventeur: **Tournier, Hervé**
**300, Rue du Riondet**
**F-74520 Valleiry (FR)**

**Schneider, Michel**
**Domaine du Moulin 34, route d'Annecy**
**CH-1256 Troinex (CH)**

**Hyacinthe, Roland**
**Aubonne**
**F-74140 Douvaine (FR)**

**Baudet, Loic**
**8, rue du Marché**
**CH-1277 Carouge (CH)**

(74) Mandataire: **Dousse, Blasco et al**
**7, route de Drize**
**CH-1227 Carouge/Genève (CH)**

(54) **Copolymère bioadhésif utilisable comme vecteur d'application de médicaments par contact direct.**

(57) Ce copolymère est composé d'acide polyacrylique ou méthacrylique et d'un mélange de mono- et diéthers insaturés de glycols ou de polyols. Sous forme solide ou liquide, il adhère aux tissus organiques en présence d'humidité. Pour l'utiliser, on y incorpore par mélange ou dissolution une proportion d'un remède et on applique ce mélange sur un tissu humide, par exemple une muqueuse, où il adhère le temps que le remède pénètre dans l'organisme par diffusion à travers les tissus.

FIG. 4

EP 0 309 404 A2

## Description

## COPOLYMERE BIOADHESIF UTILISABLE COMME VECTEUR D'APPLICATION DE MEDICAMENTS PAR CONTACT DIRECT

La présente invention a pour objet des polymères hydrophiles bioadhésifs, c'est-à-dire possédant des propriétés d'adhésion sur les tissus organiques et biologiques, notamment des organismes vivants (plantes et animaux), en présence d'humidité. L'invention concerne également des modes de préparation de tels polymères ainsi que leur utilisation pour la préparation de vecteurs d'application de remèdes et produits à action pharmacologique ou cosmétique qu'on administre par contact avec des membranes biologiques tels que l'épiderme ou les muqueuses.

L'utilisation de polymères hydrophiles bioadhésifs comme vecteurs de médicaments et produits cosmétiques n'est pas nouvelle en soi. En effet, le document US-A-4,226,848 (TEIJIN LTD) décrit une composition bioadhésive permettant de faire diffuser lentement, par contact, une préparation pharmaceutique à travers les muqueuses orales ou nasales. Cette composition comprend une matrice bioadhésive gonflable par l'eau composée de 50 à 95% en poids d'un éther de la cellulose et de 5 à environ 50% en poids d'un polymère (ou co-polymère) de l'acide acrylique (ou un sel de cet acide); le médicament à administrer est dispersé dans cette matrice. Comme type de polymère acrylique utilisable suivant cette référence, on cite un polymère d'acide acrylique réticulé par un éther allylique du saccharose dont le degré de substitution moyen est d'au moins de 3. Un tel polymère est disponible dans le commerce sous le nom de CARBOPOL (B.F. GOODRICH Chemical Co). Il est fait mention d'un tel polymère en tant que vecteur de médicaments dans les documents suivants: US-A-2,798,053; -3,074,852; - 2,909,462; - 3,330,729.

Le document WO 85/02092 (BIO-MIMETICS INC.) décrit une composition pour le relargage progressif d'un médicament dans laquelle celui-ci est mélangé avec un polymère bioadhésif formé d'un polymère carboxylique réticulé insoluble dans l'eau contenant de 0,05 à 1,5% en poids d'agent réticulant alcoylénique, ce dernier n'étant par éthérifié. Comme polymères carboxyliques, on cite les acides polyacrylique et polyéthacrylique et comme réticulants sans fonctions éther, le 3,4-dihydroxy-1,5-hexadiène et le 2,5-diméthyl-1,5-hexadiène.

De tels polymères, qui peuvent contenir, en addition, des esters d'acides alcoyléniques tels qu'acrylates et méthacrylates d'alcoyles inférieurs et moyens, sont analogues aux produits disponibles commercialement sous le nom de POLYCARBOPHIL (fabriqués par A.H. ROBINS CO, Richmond, Va, USA).

Parmi d'autres documents concernant les bioadhésifs on peut citer: US-A-4,647,599; - 3,634,584; - 3,121,043; EP-A-139,127; - 54,279; Quoique les produits bioadhésifs, connus pour le relargage progressif de médicaments par contact avec les tissus organiques présentent des qualités indiscutables, on continue à chercher à les améliorer, notamment sur le plan de la ténacité d'adhésion, de la fiabilité, de la progressivité dans le relargage des médicaments et, surtout, dans la simplicité et la versatilité de l'emploi. On cite plus particulièrement la possiblité d'utiliser le polymère comme vecteur de médicament sous des formes aussi diverses que pastilles et comprimés adhésifs, films adhésifs, solutions aqueuses adhésives (médicaments ophtalmiques), etc.

Les polymères définis à la revendication 1 constituent un pas important dans la réalisation de tels objectifs.

De préférence, les proportions en poids du composant (1) au composant (2) sont dans un fourchette de 4 : 1 à 1 : 1, mieux, de 4 : 1 à 3 : 2. De préférence, le rapport molaire entre le diéther et le monoéther du composant (2) n'est pas inférieur à 1/2, mieux, dans l'invervalle 1/2 à 2.

Comme glycols ou polyols se prêtant à la présente invention, on peut citer de manière non exhaustive, l'éthylène glycol, la glycérine, le propylène glycol, le polyéthylène glycol (par exemple le PEG-400), l'hexane-diol, le pentaérythritol, le maltose, le sortibol, le xylitol, le saccharose, le lactose, les cyclodextrines et autres di- et oligosaccharides non réducteurs. Lorsqu'on parle, à la revendication 1, d'un diéther d'un polyol (c'est-à-dire d'un composé comprenant un nombre de OH supérieur à 2), on veut dire que le degré moyen de substitution d'un tel polyol par les groupes éthers est de 2; en d'autres termes, il n'est pas nécessaire, pour réaliser l'invention, d'isoler de manière spécifique dans un mélange d'éthérification d'un polyol (comme par exemple le saccharose) les dérivés de substitution de degré 1 et 2 et de n'utiliser que ceux-ci; on peut en effet utiliser un mélange d'éthérification contenant principalement le composé disubstitué en mélange avec le monosubstitué et de faibles proportions (selon une distribution statistique) des composés polysubstitués. Ce point présente une importance non-négligeable car c'est précisément à cette difonctionalité moyenne prépondérante des monomères insaturés qu'on attribue les propriétés d'adhésion remarquable en présence d'humidité du copolymère de l'invention.

La portion insaturée des éthers faisant partie du présent copolymère est, de préférence, un groupe vinyle ou allyle. On peut, cependant, avoir d'autes groupes insaturés, par exemple isopropényle, buténlye ou isobutényle.

Le présent copolymère peut contenir, en addition, des esters hydrophobes des acides acrylique et méthacrylique, une certaine proportion de tels esters, de l'ordre de 2 à 20% en poids par exemple, ayant un effet bénéfique sur la ténacité de l'adhésion vis-à-vis des substrats à base de polyaminoacides et glycoprotéines. Comme tels esters, on peut citer les acrylates et méthacrylates d'alcoyles inférieurs, tels que éthyle, butyle, propyle, isopropyle, butyle, décyle, dodécyle, etc.

Le présent copolymère peut également contenir d'autres monomères hydrophiles tels qu'acrylamide, acrylonitrile, acrylate et méthacrylate d'hydroxyéthyle, acide maléique etc. Il est à remarquer que les propriétés du présent polymère dépendent des proportions relatives des composants (1) et (2) et peuvent être contrôlées en ajustant ces proportions de manière définie, notamment et ce qui concerne le taux de gonflement par absorption d'eau lequel augmente avec la proportion du composant (2).

Pour la préparation desdits copolymères, on procède à une copolymérisation catalysée des divers monomères en milieu aqueux. Lorsque le mélange contient, en additiion, des monomères hydrophobes, la polymérisation se fait en émulsion sui vant les moyens habituels, c'est-à-dire avec addition d'un surfactant émulsionnant. Comme catalyseurs de polymérisation, on utilise les composés connus à cet effet; de préférence on utilise les peroxydes ou persels minéraux ou organiques.

Lorsque la polymérisation est terminée, on obtient le produit sous forme d'une solution visqueuse ou d'un gel acide qu'on peut neutraliser à un degré quelconque par addition d'une base minérale ou d'amines organiques. Par solution visqueuse, on entend une suspension aqueuse de particules ou micelles d'un gel hydrophile. Généralement, on évite une telle neutralisation et on procède à la purification du produit par lavage simple à l'eau ou, mieux, par dialyse dans de l'eau pure au moyen d'une membrane de porosité appropriée pour éliminer tous les monomères n'ayant pas réagi, les polymères de bas poids moléculaire, ainsi que les catalyseurs. Puis on peut utiliser de produit tel quel en solution comme porteur de médicament, ou procéder à son séchage. Pour effectuer ce séchage, on peut déposer la solution en couche sur un substrat et laisser l'eau s'évaporer sous vide ou sous pression ordinaire, ce qui fournit des films de copolymère. En variante, on peut lyophiliser la solution ce qui fournit le produit sous forme d'une masse pulvérulente qu'on peut broyer puis comprimer en tablettes.

Pour utiliser le produit suivant l'invention comme vecteur d'administration de médicaments, on peut incorporer ceuxci au stade de solution (ou suspension de gel) ou au stade de produit sec. La méthode à choisir dépend des propriétés du médicament et des buts poursuivis en ce qui concerne le lieu et la vitesse de relargage. Si les médicaments sont hydrosolubles, on peut les incorporer à la solution (ou suspension) dialysée et employer celle-ci directement (par exemple dans le cas d'un collyre adhésif à la cornée de l'oeil); on peut aussi lyophiliser une telle solution de manière à obtenir un solide où le médicament est intimement dissous dans le copolymère; son relargage dépend alors de sa vitesse de diffusion en milieu humide lorsque le polymère ainsi chargé est appliqué sur un tissu organique, par exemple une muqueuse buccale, stomacale, intestinale ou autre; c'est en effet là une des propriétés exceptionnelles du présent polymère qui, suivant son degré de séchage, n'adhère pas instantanément à une paroi humide mais demande, pour s'y attacher, quelques instants de réhydratation. Il est donc possible d'avaler une telle tablette de manière que le polymère qui la compose se fixe à une certaine distance du point d'ingestion sur la muqueuse du tube digestif à travers laquelle le médicament est lentement relaché.

En variante, on peut bien entendu, mélanger à sec un médicament avec le présent polymère, celui-ci étant préalablement pulvérisé à une granulométrie choisie, le mélange est ensuite pressé en tablettes ou comprimés qu'on applique par pression à l'endroit voulu.

Les médicaments qu'on peut administrer de cette manière sont très nombreux et on peut citer les analgésiques, les anti-inflammatoires, les antibiotiques, les somnifères, les antidépresseurs, les tranquilisants, les hormones, les anticoagulants et autres. Une liste assez détaillée de médicaments possibles figure dans le document WO 85/02092, pages 11 et 12. On peut également utiliser les polymères de la présente invention sous forme de crèmes et lotions (incorporant les adjuvants habituels de ces produits), par exemple pour des cosmétiques et produits de beauté, colorants, écrans antisolaires, etc.

Il est à noter que lorsque le présent copolymère contient, en tant que polyol, de la cyclodextrine, cet oligomère cylique agit de manière particulièrement efficace pour le stockage et le relargage contrôlé de médicaments. En effet, de par sa structure cyclique et les fonctions hydroxyles libres qu'il comporte, il peut former, avec de nombreuses molécules actives, des complexes d'inclusion qui permettent en particulier d'améliorer leur dissolution (cas des principes actifs hydrophobes), d'augmenter dans certains cas leur biodisponibilité, de diminuer également leurs effets secondaires et éventuellement d'améliorer leur stabilité.

On prépare les éthers insaturés de glycols et de polyols par les moyens habituels en chimie, notamment par condensation entre un halogénure d'alcényle et le composé hydroxylé en milieu alcalin. Une telle technique est décrite, par exemple dans les références suivantes: P.L. Nichols Jr, and E. Yanovsky, J. Am. Chem. Soc., 67, 46 (1945) / P.L. Nichols Jr. and E. Yanovsky, Sugar, sept. 1947, 28 / M. Zief and E. Yanovsky, Industrial and Engineering Chemistry, 41, 1967, (1949) / E.L. Griffin et Col., Industrial and Engineering Chemistry, 43, 2629, (1951) / P.L. Nichols Jr. and E. Yanovsky, J. Am. Chem. Soc., 66, 1625, (1944) / R.G. Schweiger, Journal of Polymer Science A. (2), 2471, (1964).

Les exemples qui suivent illustrent l'invention.

La fig 1 représente l'application sur la muqueuse buccale d'un animal de laboratoire d'un pastille constituée d'un copolymère suivant l'invention.

La fig 2 représente l'état de cette pastille après 3-4 h.

La fig 3 est un graphique montrant le taux de relargage au cours du temps d'un médicament incorporé au polymère de l'invention.

La fig 4 est un graphique similaire à celui de la fig 3, mais concernant un autre médicament.

## Exemple 1

On a introduit dans un ballon muni d'un agitateur 278,1 g (0,81 mole) de saccharose et une solution de 66 g (1,65 moles) de NaOH dans 75 ml d'eau. On a chauffé tout en agitant jusqu'à obtention d'un sirop. A 85°C, on a introduit goutte à goutte, en 1 h, 199,2g (1,65 mole) de bromure d'allyle puis, après l'addition, on a maintenu le tout en agitation à 85°C jusqu'à obtention du pH 7 (2 h).

On a laissé refroidir et dilué par 200 ml d'eau; on a procédé à l'analyse du produit en solution par HPLC avec, comme éluant, un mélange eau/acétonitrile et, comme détecteur, un réfractomètre différentiel. Cette analyse a montré que le mélange contenait environ 60% des mono et diéthers dans un rapport pondéral d'environ 1 et moins de 30% (en moles) de composés polyéthérifiés.

On a utilisé directement cette solution pour les opérations ultérieures de la manière suivante.

On a mis en agitation et chauffé à 80°C un mélange de 144 g (2 moles) d'acide acrylique dans 150 ml d'eau avec 60 g du mélange d'éthers allyliques du saccharose en solution aqueuse préparé comme décrit ci-dessus, 2 ml de $FeCl_3$ aqueux à 5%, 2 ml de $NaHSO_3$ à 5% et 2 ml de solution aqueuse à 5% de $(NH_4)_2 S_2O_8$.

Après 3 min environ, on constate une prise du mélange sous forme d'un gel de couleur jaune qu'on a laissé reposer 1 h, dilué par 800 ml d'eau, broyé dans un moulin et soumis à une dialyse à travers une membrane de porosité Mw = 10.000. Après 24 h de dialyse dans l'eau pure courante, on a obtenu une masse incolore gélifiée qu'on a lyophilisée: rendement 126 g de solide granuleux incolore qu'on a ensuite broyé à sec en une poudre fine.

On a pressé cette poudre sous 750 - 1000 bar pour en constituer des pastilles de 13 mm de diamètre et de 0,2 - 0,5 mm d'épaisseur. Ces pastilles, à l'état sec, n'adhèrent pas aux doigts; par contre, après les avoir mises en contact 1 - 2 secondes avec un papier filtre humide, elles collent fortement aux doigts, au point qu'il est difficile de les arracher.

## Exemple 2

On a utilisé le polymère sec broyé décrit à l'exemple 1 et on l'a mélangé à 30% en poids d'un médicament absorbable par les muqueuses, puis on a pressé des pastilles comme décrit à l'exemple précédent.

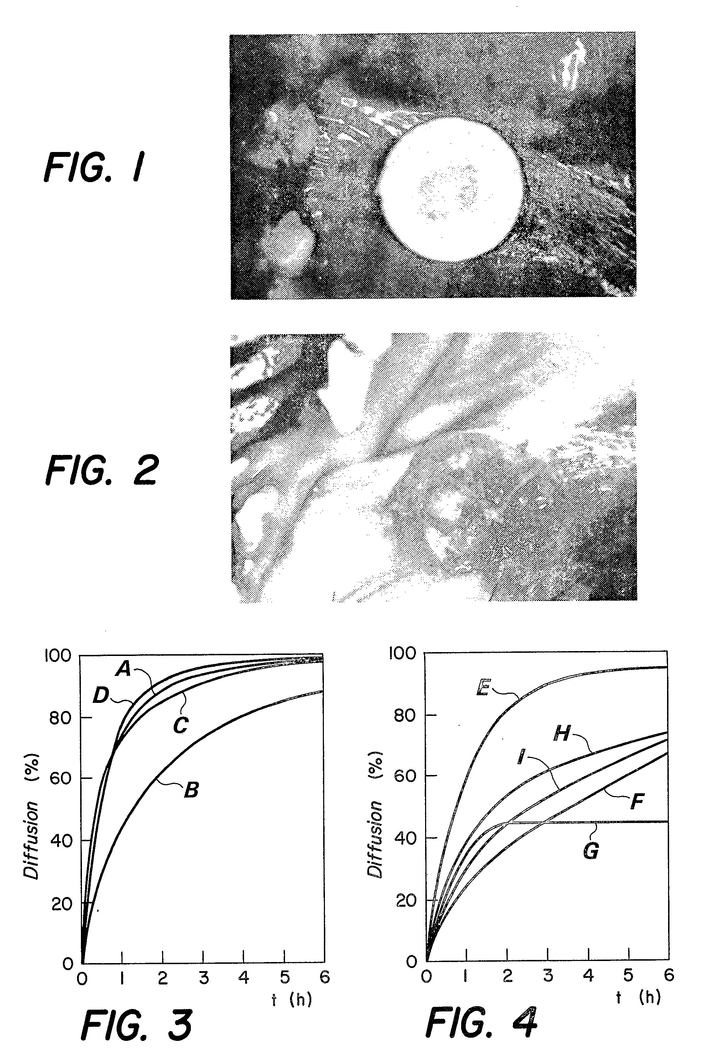

On a appliqué ces pastilles sur la muqueuse buccale de chiens de laboratoires. La fig 1 représente une de ces pastilles fraîchement appliquée, c'est-à-dire au temps zéro. La fig 2 montre la même pastille après 4 h d'attente et après que le chien ait eu son repas. On constate que l'adhésion est parfaite et que la pastille se dégrade peu à peu comme prévu, ce qui permet au médicament d'être progressivement relargué.

## Exemple 3

On a répété le processus de formation de copolymère tel que décrit à l'exemple 1, avec cependant la variation suivante: en plus des ingrédients cités, on a ajouté 20 g d'acrylate de dodécyle et, ce produit étant insoluble dans l'eau, une goutte d'un surfactant (Tween), de manière à réaliser, par agitation, une émulsion très fine.

La masse de polymère obtenue par cette réaction en émulsion ne présente pas de différences fondamentales visibles avec celle décrite à l'exemple 1; elle est cependant plus compacte. On a procédé à sa purification et à son séchage comme déjà décrit. On a constaté que son pouvoir d'adhésion sur les muqueuses humides était similaire à celui du produit de l'exemple 1.

## Exemple 4

On a procédé comme décrit à l'exemple 1, mais en remplaçant le polyol saccharose par du polyéthylène glycol 400 (PEG 400) en proportions molaires équivalentes. L'analyse par HPLC du produit d'éthérification par le bromure d'allyle a montré que le mélange contenait, outre un peu de PEG n'ayant pas réagi, du diéther et du monoéther en proportion molaire approximativement 2 : 1.

On a utilisé ce mélange pour une copolymérisation avec l'acide acrylique au taux de 30 parties en poids d'éthers allyliques pour 70 parties de monomère acide acrylique et 7 parties d'acrylamide. On a ainsi obtenu une solution visqueuse formant un film adhésif très souple après séchage. On peut aussi utiliser cette préparation en mélange avec les excipients ophtalmologiques pour préparer un collyre qui n'est que lentement éliminé après instillation dans les yeux.

## Exemple 5

On a procédé à des essais de diffusion d'un médicament relargué par le copolymère de l'invention à travers une membrane de cellulose régénérée.

Pour cela, on a utilisé une solution aqueuse de théophylline à 5 g/l. A une prise de 10 ml de cette solution (A), on a ajouté 25 mg de polymère tel que décrit à l'exemple 1 et 25 mg du même copolymère dont les fonctions COOH ont été salifiées par la soude et on a placé le mélange de solution A et des deux compolymères (mélange dénommé B) dans une enveloppe de dialyse, elle même placée en contact avec de l'eau dans un appareil prévu à cet effet (Type SOTAX AT-6 normalisé, à 37°C, 100 r.p.m.). Des échantillons de contrôle similaires ont été réalisés en utilisant une quantité identique de solution (A) et, en mélange, 50 mg de polymères connus, à savoir le CARBO-POL-934 commercial (mélange dénommé C) d'une part, et un polymère de type POLYCABOPHIL No 1 préparé au laboratoire par polymérisation et réticulation de l'acide acrylique par du 2,5-diméthyl-1,5-hexadiène (mélange dénommé D), d'autre part. On a églament constitué un témoin par la solution (A) seule. On a ensuite mesuré , par spectrophotométrie à $\lambda = 269$ nm, le relargage de la théophylline au cours du temps dans les quatre cas: A, la théophylline seule, B, le polymère de l'invention, C et D les polymères connus témoins.

Les résultats sont consignés au graphique de la fig 3 où on constate, par les valeurs de % de diffusion en fonction du temps, que les courbes (A), (C) et (D), presque confondues, traduisent un relargage beaucoup plus rapide et moins régulier que (B) le polymère de l'invention.

### Exemple 6

On a procédé comme décrit à l'exemple 5 pour étudier la diffusion de la cimétidine. La courbe E de la fig 4 correspond à une solution aqueuse à 5 g/l de cimétidine et les courbes suivantes correspondent à 10 ml de cette solution additionnée de 50 mg des polymères suivants:

    F: polymère suivant l'exemple 1 salifié par l'ion Na$^+$

    G: CARBOPOL-934

    H: POLYCARBOPHIL No 1 (voir l'exemple précédent)

    I: POLYCARBOPHIL No 3 (il s'agit-là d'un polymère de type POLYCARBOPHIL préparé dans nos laboratoires à partir d'acide acrylique réticulé par du divinyl-glycol)

On voit également dans cet exemple que le polymère de l'invention relâche le médicament plus régulièrement et plus lentement que les produits correspondants de l'état de la technique.

### Exemple 7

On a préparé suivant les directives du fabricant une suspension d'oxychlorure de cuivre à 1,5% destiné à lutter contre les maladies fongiques des végétaux. A cette suspension, on a ajouté 1% polymère préparé suivant l'exemple 7 et on l'a homogénéisé de manière qu'il se disperse finement dans la préparation de cuivre.

On a ensuite pulvérisé ce mélange sur des végétaux susceptibles d'être attaqués par des maladies fongiques et on a constaté que la préparation ainsi modifiée adhérait plus longtemps à ces végétaux qu'une préparation témoin non modifiée.

## Revendications

1. Copolymère hydrophile bioadhésif utilisable comme vecteur-contact pour l'application et le relargage progressif de médicaments à travers les tissus organiques, notamment biologiques, humides, caractérisé en ce qu'il est constitué d'un copolymère de (1) l'acide acrylique et/ou méthacrylique et (2) des mono- et diéthers insaturés d'un glycol ou d'un polyol, les proportions pondérales des composants (1) et (2) étant de 20 : 1 à 1 : 1.

2. Copolymère suivant la revendication 1, caractérisé en ce que le rapport molaire entre le diéther et le monoéther du composant (2) est compris entre 1 : 2 et 2 : 1.

3. Copolymère suivant la revendication 1, caractérisé en ce qu'il contient encore des éthers insaturés de polyols d'un degré de substitution supérieur à 2, le rapport pondéral entre ceux-ci et les dérivés mono- et di-substitués ne dépassant pas 30%.

4. Copolymère suivant les revendications 1 à 3, caractérisé en ce qu'il contient, en addition, de 2 à 20% en poids d'un ou plusieurs acrylates ou méthacrylates hydrophobes, par exemple des esters d'alcoyles en $C_2$ à $C_{12}$.

5. Copolymère suivant les revendications 1 à 4, caractérisé en ce que les glycols et polyols sont choisis parmi: éthylène glycol, propylène glycol, butylène glycol, polyéthylène glycol, glycérol, pentaérythritol, saccharose, sorbitol, xylitol, lactose, cyclodextrines et autres.

6. Copolymère suivant les revendications 1 à 5, caractérisé en ce que les éthers insaturés sont des éthers vinyliques ou allyliques.

7. Procédé de préparation des copolymères suivant la revendication 1, caractérisé en ce qu'on copolymérise en milieu aqueux, en présence de catalyseurs de polyméristion oléfinique, un monomère acide acrylique ou méthacrylique en mélange avec des mono- et diéthers insaturés de glycols ou polyols, ces éthers étant obtenus par traitement desdits glycols ou polyols par deux équivalents d'halogènure d'alcényle en milieu alcalin aqueux.

8. Procédé suivant la revendication 7, dans lequel le mélange de monomère contient, en plus, un acrylate hydrophobe, caractérisé en ce qu'on opère en émulsion aqueuse en présence d'un surfactant.

9. Procédé suivant la revendication 8, caractérisé en ce qu'après la polymérisation, on purifie le copolymère par lavage à l'eau ou par dialyse dans l'eau avec une membrane de porosité contrôlée,

10. Procédé suivant la revendication 9, caractérisé en ce qu'après purification, on sépare le polymère à l'état solide par évaporation ou lyophilisation.

11. Procédé suivant la revendication 7, caractérisé en ce qu'après la polymérisation on neutralise partiellement ou totalement les fonctions carboxyliques acides par une base minérale ou organique de façon à obtenir des sels.

12. Utilisation du copolymère suivant la revendication 1, comme vecteur pour administrer à un organisme vivant des principes actifs par contact avec les tissus de cet organisme, caractérisée en ce qu'on mélange ce principe actif avec ledit polymère et qu'on applique ce mélange sur ledit tissu en présence d'humidité, les propriétés bioadhésives du polymère maintenant celui-ci au lieu d'application, ou dans son voisinage immédiat, le temps que le principe actif se désorbe et pénètre dans l'organisme par diffusion.

13. Utilisation suivant la revendication 12, caractérisée en ce que le principe actif est un médicament, que le mélange est solide, qu'on le forme par pression en comprimés, pastilles ou tablettes et qu'on applique celles-ci sur une muqueuse humide où il adhère parfaitement.

14. Utilisation suivant la revendication 12, caractérisée en ce que le mélange est liquide, de manière qu'à l'application il s'étale en formant un film qui adhère sur la zone d'application.

15. Utilisation suivant la revendication 12, caractérisée en ce que le polymère est, au moins en partie, sous forme d'un sel de cations minéraux ou organiques, la progressivité et la régularité de son pouvoir de relargage du médicament au cours du temps étant, sous cette forme, améliorées.

16. Utilisation suivant la revendication 12, caractérisée en ce que l'application concerne les muqueuses buccales, nasales, vaginales, pulmonaires, ainsi que celles du système digestif, les yeux, les oreilles et la peau.

17. Utilisation suivant la revendication 12, caractérisée en ce que le principe actif est un produit de traitement phytosanitaire.

FIG. 1

FIG. 2

FIG. 3

FIG. 4